# EUROPÄISCHE PATENTSCHRIFT

(11) **EP 3 517 579 B1**
(45) Veröffentlichungstag und Bekanntmachung des Hinweises auf die Patenterteilung: **26.10.2022**
(21) Anmeldenummer: 19152699.5
(22) Anmeldetag: 21.01.2019
(51) Int. Cl.: C09C 1/00, A61K 8/26, A61Q 1/02, A61K 8/02, A61K 8/19, A61K 8/25, A61K 8/29, A61Q 1/12

(54) **PIGMENTGEMISCH ENTHALTEND ROTE, BLAUE UND GRÜNE INTERFERENZPIGMENTE**
PIGMENT MIXTURE COMPRISING RED, BLUE AND GREEN INTERFERENCE PIGMENTS
MÉLANGE DE PIGMENTS INTERFÉRENTIELS ROUGE, BLEU ET VERT

(30) Priorität: 23.01.2018 EP 18153057
(43) Veröffentlichungstag der Anmeldung: 31.07.2019
(73) Patentinhaber: Merck Patent GmbH, 64293 Darmstadt (DE)
(72) Erfinder: Schmidt, Christoph, 65830 KRIFTEL (DE); Plueg, Carsten, 64367 MUEHLTAL (DE); Lenz, Uwe, 64673 Zwingenberg (DE); Schoen, Sabine, 45701 HERTEN (DE)

(56) Entgegenhaltungen:
- WO-A2-2007/031970
- JP-A- 2014 189 514
- US-A1- 2014 271 740

## Beschreibung

Die vorliegende Erfindung betrifft ein Pigmentgemisch enthaltend mindestens zwei oder drei Interferenzpigmente unterschiedlicher Interferenzfarben ausgewählt aus den Farben Rot, Grün und Blau, sowie dessen Verwendung in Farben, Lacken, Druckfarben, Sicherheitsdruckfarben, Kunststoffen, keramischen Materialien, Gläsern und zur Herstellung von Pigmentpräparationen und Trockenpräparaten und insbesondere in kosmetischen Formulierungen.

In der dekorativen Kosmetik ist die optische Neutralisierung von Hautunregelmäßigkeiten und/oder unerwüschten Farbnuancen der Haut ein wichtiges Anwendungsgebiet. Ziel ist es dabei der Haut, insbesondere im Gesichtsbereich, ein gleichmäßiges Aussehen und einen individuell gewünschten Farbton zu verleihen ohne das natürliche Erscheinungsbild zu mindern.

Vielfach werden hierfür klassische Absorptionspigmente verwendet, die jedoch den Nachteil haben, die Haut maskenhaft zu verdecken.

In der WO 2007/031970 werden Hautpflegezusammensetzungen beschrieben, die Interferenzpigmente enthalten, welche paarweise komplementäre Farben besitzen. Durch die Verwendung von komplementären Pigmentpaaren kann nur die Farbintensität entlang einer Farbkurve in einem sehr engen Bereich gesteuert werden. Um aber den erforderlichen Farbbereich für die Verwendung auf der Haut abzudecken, lehrt die WO 2007/031970 zudem, dass vier Farbpigmente in Form von zwei komplementären Farbpaaren Rot-Grün und Blau-Gelb nötig sind. Diese Vorgehensweise hat den Nachteil, dass die Farbe der Formulierung nicht frei nach Wunsch eingestellt werden kann. Insbesondere die gegenseitige Farblöschung der komplementären Farbpaare macht die Einstellung einer bestimmten Farbe und Farbintensität für einen bestimmten Hauttyp sehr schwierig oder gar unmöglich.

Aufgabe der vorliegenden Erfindung ist es daher ein System aus wenigen Pigmenten zu finden, das es ermöglicht über einen weiten Farbtonbereich jeden individuell gewünschten Farbton sowie jede Farbintensität einzustellen, so dass beispielsweise in der Kosmetik Hautunregelmäßigkeiten neutralisiert werden können, ohne dass durch Farblöschung die Brillianz des Erscheinungsbildes beeinträchtigt wird.

Überraschenderweise wurde gefunden, dass durch die Verwendung farblich besonders geeigneter roter, grüner und blauer Interferenzpigmente, die Zahl der erforderlichen Interferenzfarben auf zwei oder drei reduziert werden kann und gleichzeitig die Bandbreite der erzielbaren Farbtöne und Farbintensitäten gegenüber dem Stand der Technik deutlich erhöht wird und individuell eingestellt werden kann. Hierdurch kann die kosmetische Formulierung in einfacher Weise jedem Hauttyp angepasst werden.

Gegenstand der vorliegenden Erfindung ist somit gemäß Anspruch 1 ein Pigmentgemisch enthaltend zwei oder drei Interferenzpigmente, die jeweils unterschiedliche Interferenzfarben aufweisen, wobei die Interferenzfarben ausgewählt sind aus den Farben Rot, Blau und Grün und im CIE-L*a*b*-Farbbereich wie folgt definiert sind:

| | |
|---|---|
| rotes Interferenzpigment: | a ≥ 25 und b ≥ 0, |
| blaues Interferenzpigment: | -20 ≤ a ≤ 40 und b ≤ -50 |
| grünes Interferenzpigment: | a ≤ -15 und b ≥ 0. |

Gegenstand der Erfindung ist weiterhin die Verwendung des erfindungsgemäßen Pigmentgemisches in Farben, Lacken, vorzugsweise in Industrie- und Automobillacken, Druckfarben, Sicherheitsdruckfarben, Kunststoffen, keramischen Materialien, Gläsern, als Tracer, als Füllstoff und insbesondere in kosmetischen Formulierungen. Weiterhin sind die erfindungsgemäßen Pigmente auch zur Herstellung von Pigmentpräparationen sowie zur Herstellung von Trockenpräparaten, wie z. B. Granulaten, Pearlets, Chips, Pellets, Würstchen, Briketts, etc., geeignet. Die Trockenpräparate finden insbesondere Anwendung in Druckfarben und in der Kosmetik.

Die geeigneten Interferenzpigmente sind gekennzeichnet durch die folgenden Eigenschaften im CIE-L*a*b*-Farbbereich:
Das rote Interferenzpigment weist im CIE-L*a*b*-Farbbereich folgende a- und b-Werte auf:
a ≥ 25 und b ≥ 0, vorzugsweise a ≥ 30 und b ≥ 5, insbesondere a ≥ 35 und b ≥ 5.

Das blaue Interferenzpigment weist im CIE-L*a*b*-Farbbereich folgende a- und b-Werte auf:
-20 ≤ a ≤ 40 und b ≤ -50, vorzugsweise -15 ≤ a ≤ 30 und b ≤ -60, insbesondere -10 ≤ a ≤ 20 und b < -65.

Das grüne Interferenzpigment weist im CIE-L*a*b*-Farbbereich folgende a- und b-Werte auf:
a ≤ -15 und b ≥ 0, vorzugsweise a ≤ -20 und b ≥ 15, insbesondere a ≤ -25 und b ≥ 20.

Das erfindungsgemäße Pigmentgemisch kann
- ein rotes und ein grünes Interferenzpigment oder
- ein rotes und ein blaues Interferenzpigment oder
- ein grünes und ein blaues Interferenzpigment oder
- ein rotes, blaues und grünes Interferenzpigment
enthalten, wobei die Interferenzpigmente mit ihren Interferenzfarben, die oben angegebenen Bedingungen für die a- und b-Werte erfüllen müssen.

Die vorliegende Erfindung ermöglicht die Realisierung von goldenen Farbtönen mit einem roten, grünen und/oder blauen Effektpigment. Ein goldfarbenes Effektpigment ist daher nicht notwendig, kann aber zur Erzielung besonders gewünschter Farbtöne zusätzlich verwendet werden.

Bevorzugte Gemische aus Interferenzpigmenten enthaltend mindestens zwei Interferenzpigmente aus der Gruppe rotes, grünes und blaues Interferenzpigment, sind solche, bei denen die Einzelpigmente die folgenden CIE-L*a*b*-Farbbereiche erfüllen:
rotes Interferenzpigment: a ≥ 30 und b ≥ 5, blaues Interferenzpigment - 15 ≤ a ≤ 30 und b < -60, grünes Interferenzpigment: a ≤ -20 und b ≥ 15.

Ganz besonders bevorzugte Pigmentgemische enthalten mindestens zwei Interferenzpigmente, die in folgende CIE-L*a*b*-Farbbereiche fallen:

| | |
|---|---|
| rotes Interferenzpigment: | a ≥ 35 und b ≥ 5, |
| blaues Interferenzpigment: | -10 ≤ a ≤ 20 und b < -65 und/oder grünes |
| Interferenzpigment: | a ≤ -25 und b ≥ 20. |

Die roten, grünen und blauen Interferenzpigmente können in Abhängigkeit der gewünschten Farbe und Farbintensität in jedem Verhältnis miteinander gemischt werden.

Bevorzugte Mischungsverhältnisse (= Mengenverhältnisse) der roten, grünen und/oder blauen Interferenzpigmente werden nachfolgend genannt: rotes Interferenzpigment + grünes Interferenzpigment: 7 : 3 und kleiner, vorzugsweise 3 : 7, insbesondere 1 : 1;
rotes Interferenzpigment + blaues Interferenzpigment: 2 : 1 und größer, vorzugsweise 10 : 1 insbesondere 20 : 1;
blaues Interferenzpigment + grünes Interferenzpigment: 1 : 2 und kleiner, vorzugsweise 1 : 10, insbesondere 1 : 20;
rotes Interferenzpigment + grünes Interferenzpigment + blaues Interferenzpigment: 2 : 2 : 1 und größer, vorzugsweise 5 : 5 : 1, insbesondere 10 : 10 : 1

Durch das gewählte Mengenverhältnis der zwei bzw. drei Interferenzpigmente kann die Farbintensität und der Farbton der vier Farben Rot/Grün/Blau/Gold des Pigmentgemisches gezielt eingestellt werden.

Interferenzpigmente sind in der Literatur vielfach beschrieben und dem Fachmann bekannt. Interferenzpigmente, die für das erfindunsgegemäße Pigmentgemisch insbesondere geeignet sind, basieren auf plättchenförmigen Substraten. Geeignete plättchenförmige Substrate für die Interferenzpigmente sind einerseits opake und andererseits transparente plättchenförmige Substrate. Bevorzugte plättchenförmige Substrate sind Schichtsilikate. Insbesondere geeignet sind natürlicher oder synthetischer Glimmer, Talkum, Kaolin, plättchenförmige Fe₂O₃-, Fe₃O₄-, Al₂O₃-, BiOCl-, Glas-, SiO₂-, TiO₂-, BN-, Oxinitrid-, Nitrid-, Graphitplättchen, Fischsilber, synthetische trägerfreie Plättchen oder andere vergleichbare Materialien. Bei dem erfindungsgemäßen Pgimentgemisch können auch Interferenzpigmente verwendet werden, die auf unterschiedlichen Substraten basieren.

Besonders bevorzugte Interferrenzpigmentgemische basieren auf Glimmerplättchen + SiO₂-Plättchen
Glimmerplättchen + Al₂O₃-Plättchen
Glimmerplättchen + Glas-Plättchen
Glimmerplättchen + TiO₂-Plättchen
Glimmerplättchen + Oxinitrid-Plättchen
Glimmerplättchen + Nitrid-Plättchen
Glimmerplättchen + Fischsilber
Glimmerplättchen + Graphitplättchen
Glimmerplättchen + BiOCI
SiO₂-Plättchen + Al₂O₃-Plättchen
Glasplättchen + SiO₂-Plättchen.

Insbesondere bevorzugt sind Interferenzpigmente, die auf natürlichen Glimmerplättchen, synthetischen Glimmerplättchen, beschichteten und unbeschichteten Al₂O₃-Plättchen, SiO₂-Plättchen und Glasplättchen basieren. Bei den beschichteten Glasplättchen handelt es sich vorzugsweise um Glasplättchen, die mit einer SiO₂-Schicht belegt sind. Die Glimmerplättchen können ebenfalls vor der Belegung mit der Interferenzschicht mit einer dünnen Schicht (< 10 nm) aus SiO₂ oder Al₂O₃ belegt werden.

Sofern die Interferenzpigmente auf Al₂O₃-Plättchen basieren, handelt sich sich vorzugsweise um alpha-Aluminiumoxid. Die Al₂O₃-Plättchen können dotiert oder undotiert vorliegen. Sofern sie dotiert sind, ist das Dotiermittel vorzugsweise ausgewählt aus der Gruppe TiO₂, ZrO₂, SiO₂, In₂O₃, SnO₂, und ZnO sowie Kombinationen davon. Der Anteil der Dotierung bezogen auf das Aluminiumoxid beträgt vorzugsweise 0,01-5 Gew.%. In einer bevorzugten Ausführungsform enthält das Alumniumoxidplättchen lediglich ein Dotiermittel, wobei es sich insbesondere um TiO₂, ZnO oder ZrO₂ handelt.

Die Größe der plättchenförmigen Substrate ist an sich nicht kritisch und kann auf den jeweiligen Anwendungszweck abgestimmt werden. In der Regel haben die plättchenförmigen Substrate eine Dicke zwischen 0,05 und 1,5 µm, insbesondere zwischen 0,1 und 1 µm. Die Ausdehnung in den beiden anderen Bereichen beträgt üblicherweise zwischen 1 und 250 µm, vorzugsweise zwischen 2 und 200 µm, und insbesondere zwischen 5 und 60 µm. Es können auch Substrate unterschiedlicher Partikelgrößen eingesetzt werden. Besonders bevorzugt ist ein Gemisch aus Glimmerfraktionen von N-Glimmer (10-60 µm), F-Glimmer (5-20 µm) und/oder M-Glimmer (<15 µm). Weiterhin bevorzugt sind N- und S-Fraktionen (10-130 µm)und F- und S-Fraktionen (5-130 µm).

Interferenzpigmente, die für das erfindungsgemäße Pigmentgemisch insbesondere geeignet sind, basieren auf plättchenförmigen Substraten, die mit ein oder mehreren Interferenzschichten, vorzugsweise ein oder mehrere Metalloxide, auf der Oberfläche beschichtet, d. h. vollständig umhüllt, sind. Besonders bevorzugt sind Interferenzpigmente, die auf plättchenförmigen Substraten basieren und auf der Oberfläche eine Beschichtung aus TiO₂ und/oder Fe₂O₃ oder eine Schichtenfolge unter Verwendung von zwei oder mehreren Oxiden aus der Reihe von z.B. TiO₂, SiO₂, Fe₂O₃, Cr₂O₃, ZrO₂, Al₂O₃, SnO₂, aufweisen, wobei mindestens ein Oxid in der Schichtenfolge hochbrechend sein sollte und einen Brechungsindex von ≥ 1,8 aufweisen sollte.

Sofern die Interferenzschicht aus TiO₂ besteht oder TiO₂ enthält, kann das Titandioxid in der Rutil- oder in der Anatasmodifikation vorliegen. Vorzugsweise liegt das Titandioxid als Rutil vor.

Besonders bevorzugt sind Interferenzpigmente mit roten, grünen und blauen Interferenzfarben, die eine Partikelgröße von 2 - 70 µm, bevorzugt 10 - 60 µm, und bevorzugt 4-28 µm (F-Fraktion) aufweisen und als Substrat natürlichen oder synthetischen Glimmer enthalten. Bevorzugt sind weiterhin rote und grüne Interferenzpigmente, die einen gelbstichigen Farbton aufweisen. Dieser "Gelbstich" spiegelt sich im CIE-L*a*b*-Farbbereich in dem b-Wert wieder, d.h. der b-Wert muss > 0 sein. Durch das Mischen eines definierten roten Interferenzpigmentes mit einem definierten grünen Interferenzpigment jeweils mit einem Gelbstich ist es möglich Rot-, Grün- und Goldtöne verschiedener Farbtönung und Farbintensität einzustellen.

Um in den Anwendungen, vorzugsweise in den kosmetischen Formulierungen, alle Farbtöne abzudecken, empfiehlt es sich weiterhin die Partikelgrößen sowie die Partikelgrößenverteilung der Interferenzpigmente im erfindungsgemäßen Pigmentgemisch einzustellen.

Die roten Interferenzpigmente besitzen vorzugsweise eine Partikelgrößenverteilung (bestimmt nach Malvern, Mastersizer 2000) von
D₁₀ = < 12 µm, vorzugsweise < 10 µm, insbesondere < 7 µm
D₅₀ = < 25 µm, vorzugsweise < 15 µm, insbesondere < 12 µm
D₉₀ = < 45 µm, vorzugsweise < 20 µm, insbesondere < 17 µm.

Die grünen Interferenzpigmente besitzen vorzugsweise eine Partikelgrößenverteilung (bestimmt nach Malvern) von
D₁₀ = < 12 µm, vorzugsweise < 10 µm, insbesondere < 7 µm
D₅₀ = < 25 µm, vorzugsweise < 15 µm, insbesondere < 12 µm
D₉₀ = < 45 µm, vorzugsweise < 20 µm, insbesondere < 17 µm.

Die blauen Interferenzpigmente besitzen vorzugsweise eine Partikelgrößenverteilung (bestimmt nach Malvern) von
D₁₀ = < 12 µm, vorzugsweise < 10 µm, insbesondere < 7 µm
D₅₀ = < 25 µm, vorzugsweise < 15 µm, insbesondere < 12 µm
D₉₀ = < 45 µm, vorzugsweise < 20 µm, insbesondere < 17 µm.

Ganz besonders bevorzugte Interferenzpigmente besitzen eine Partikelgröße im Bereich von 5 bis 30 µm, bei einem D₉₀-Wert von < 25 µm, einem D₅₀-Wert von < 15 µm. Der D₁₀-Wert beträgt vorzugsweise < 10 µm.

Zur weiteren Verbesserung des Ergebnisses können der kosmetischen Formulierung wahlweise zusätzliche Silberinterfrenzpigmente und/oder Absorptionspigmente zugesetzt werden.

Gegenstand der Erfindung ist auch ein Verfahren zur Herstellung des erfindungsgemäßen Pigmentgemisches, welches sich dadurch auszeichnet, dass zwei oder drei Interferenzpigmente mit unterschiedlichen Interferenzfarben und definierten a- und b-Werten im CIE L*a*b*-Farbsystem miteinander gemischt werden.

Geeignete rote, grüne und blaue Interferenzpigmente mit den definierten CIE-L*a*b*-Farbwerten lassen sich leicht herstellen durch die Belegung mit einer oder mehreren hochbrechenden Interferenzschicht(en), vorzugsweise Metalloxidschicht(en), auf der Oberfläche der plättchenförmigen Substrate.

Die Metalloxidschicht(en) werden vorzugsweise naßchemisch aufgebracht, wobei die zur Herstellung von Perlglanzpigmenten entwickelten naßchemischen Beschichtungsverfahren angewendet werden können; Derartige Verfahren sind z. B. beschrieben in U.S. 3087828, U.S. 3087829, U.S. 3553001, DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017, DE 196 18 568, EP 0 659 843, oder auch in weiteren dem Fachmann bekannten Patentdokumenten und sonstigen Publikationen.

Für das Aufbringen einer SiO₂-Schicht wird bevorzugt das in der DE 196 18 569 beschriebene Verfahren verwendet. Zur Herstellung der SiO₂-Schicht wird vorzugsweise Natrium- oder Kaliumwasserglaslösung eingesetzt.

Bei der Naßbeschichtung werden die Substratplättchen in Wasser suspendiert und mit einem oder mehreren hydrolysierbaren Metallsalzen bei einem für die Hydrolyse geeigneten pH-Wert versetzt, der so gewählt wird, daß die Metalloxide bzw. Metalloxidhydrate direkt auf den Plättchen ausgefällt werden, ohne daß es zu Nebenfällungen kommt. Der pH-Wert wird üblicherweise durch gleichzeitiges Zudosieren einer Base und/oder Säure konstant gehalten. Anschließend werden die Pigmente abgetrennt, gewaschen und getrocknet und gegebenenfalls geglüht, wobei die Glühtemperatur im Hinblick auf die jeweils vorliegende Beschichtung optimiert werden kann. In der Regel liegen die Glühtemperaturen zwischen 250 und 1000 °C, vorzugsweise zwischen 350 und 900 °C. Falls gewünscht können die Pigmente nach Aufbringen einzelner Beschichtungen abgetrennt, getrocknet und ggf. geglüht werden, um dann zur Auffällung der weiteren Schichten wieder resuspendiert zu werden.

Weiterhin kann die Beschichtung auch in einem Wirbelbettreaktor durch Gasphasenbeschichtung erfolgen, wobei z.B. die in EP 0 045 851 und EP 0 106 235 zur Herstellung von Perlglanzpigmenten vorgeschlagenen Verfahren entsprechend angewendet werden können.

Der Farbton der Interferenzpigmente kann in weiten Grenzen durch unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden

Zur Erhöhung der Licht-, Wasser- und Wetterstabilität empfiehlt es sich häufig, in Abhängigkeit vom Einsatzgebiet, das Pigmentgemisch einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung wird die chemische und photochemische Stabilität weiter erhöht oder die Handhabung des Pigmentgemisches, insbesondere die Einarbeitung in unterschiedliche Medien erleichtert. Zur Verbesserung der Benetzbarkeit, Dispergierbarkeit und/oder Verträglichkeit mit den Anwendermedien können funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjee, Philips Technical Review, Vol. 44, No. 3, 81 ff. und P.H. Harding J.C. Berg, J. Adhesion Sci. Technol. Vol. 11 No. 4, S. 471-493.

Unter Beschichtung(en) sind in dieser Patentanmeldung die vollständige Belegung/Umhüllung der plättchenförmigen Substrate zu verstehen.

Das erfindungsgemäße Pigmentgemisch ist mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegwerk, GSB-Wahl, Follmann, Ruco oder Coates Screen INKS GmbH vertrieben werden. Die Druckfarben können auf Wasserbasis oder Lösemittelbasis aufgebaut sein.

Das erfindungsgemäße Pigmentgemisch ist insbesondere geeignet für die dekorative Kosmetik und für Personal Care Anwendungen, wie z.B. Nagellacke, Lippenstifte, Presspuder, Gele, Lotionen, Seifen, Zahnpasta, Body Lotions, Emulsionen, Shampoos, BB Cremes, CC Cremes, Make Up, Foundations, Mascara, Haar-, Wimpern- und Augenbrauenprodukte, etc., aber auch in Lacken, in Industrielacken und Pulverlacken, sowie in Kunststoffen und in der Keramik.

In der dekorativen Kosmetik wird das erfindungsgemäße Pigmentgemisch vorzugsweise in Konzentrationen von 0,5 - 25 Gew.%, insbesondere 1 - 20 Gew.%, und ganz besonders bevorzugt von 1 - 10 Gew.%, bezogen auf die Formulierung eingesetzt. Bei den kosmetischen Formulierungen für Personal Care Anwendungen wird das erfindungsgemäße Pigmentgemisch vorzugsweise in Konzentrationen von 0,1 - 5 Gew.%, und ganz besonders bevorzugt von 0,5 - 4 Gew.%, bezogen auf die Formulierung eingesetzt.

Da das erfindungsgemäße Pigmentgemisch es ermöglicht jede Farbe für jeden Hauttyp einzustellen, ist das Pigmentgemisch insbesondere geeignet um Hautunregelmäßigkeiten zu neutralisieren und kann daher vorzugsweise Verwendung finden in medizinische Abdecksalben, Konturstiften, Make-ups, Foundations, Concealers. Die kosmetischen Formulierungen für den Einsatz als Skin-Corrector enthalten das erfindungsgemäße Pigmentgemisch vorzugsweise in Mengen von 0,5 - 25 Gew.%, insbesondere 1 - 20 Gew.%, bezogen auf die Formulierung.

Es versteht sich von selbst, dass für die verschiedenen Anwendungszwecke das erfindungsgemäße Pigmentgemisch auch vorteilhaft in Abmischung mit z. B.
- Metalleffektpigmenten, z. B. auf der Basis von Eisen- oder Aluminiumplättchen;
- Absorptionspigmenten;
- Mehrschichteffektpigmenten (enthaltend vorzugsweise 2, 3, 4, 5 oder 7 Schichten) auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- organischen Farbstoffen;
- organischen Pigmenten;
- anorganischen Pigmenten, wie z.B. transparenten und deckenden Weiß-, Bunt- und Schwarzpigmenten;
- plättchenförmigen Eisenoxiden;
- Ruß
eingesetzt werden kann.

Das erfindungsgemäße Pigmentgemisch kann in jedem Verhältnis mit handelsüblichen Pigmenten und/oder weiteren handelsüblichen Füllstoffen gemischt werden.

Als handelsübliche Füllstoffe sind z.B. zu nennen natürlicher und synthetischer Glimmer, Nylon Powder, reine oder gefüllte Melaminharze, Talkum, Gläser, Kaolin, Oxide oder Hydroxide von Aluminium, Magnesium, Calcium, Zink, BiOCI, Bariumsulfat, Calciumsulfat, Calciumcarbonat, Magnesiumcarbonat, Kohlenstoff, Bornitrid sowie physikalische oder chemische Kombinationen dieser Stoffe. Bezüglich der Partikelform des Füllstoffes gibt es keine Einschränkungen. Sie kann den Anforderungen gemäß z.B. plättchenförmig, sphärisch oder nadelförmig sein.

Selbstverständlich kann das erfindungsgemäße Pigmentgemisch in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe, wie z.B. Bentonite, Hektorite, Siliziumdioxide, Ca-Silicate, Gelatinen, hochmolekulare Kohlenhydrate und/oder oberflächenaktive Hilfsmittel, etc.

Die das erfindungsgemäße Pigmentgemisch enthaltende Formulierung kann dem lipophilen, hydrophilen oder hydrophoben Typ angehören. Bei heterogenen Formulierungen mit diskreten wässrigen und nichtwässrigen Phasen kann das erfindungsgemäße Pigmentgemisch in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 4 und 10 liegen. Den Konzentrationen des erfindungsgemäßen Pigmentgemisches in der Formulierung sind keine Grenzen gesetzt. Sie können - je nach Anwendungsfall - zwischen 0,001 (rinse-off-Produkte, z.B. Duschgele) und 60 % liegen. Das erfindungsgemäße Pigmentgemisch kann weiterhin auch mit kosmetischen Wirkstoffen kombiniert werden. Geeignete Wirkstoffe sind z.B. Insect Repellents, anorganische UV-Filter, wie z.B. TiO₂, UV A/BC-Schutzfilter (z.B. OMC, B3, MBC), auch in verkapselter Form, Anti-Ageing-Wirkstoffe, Vitamine und deren Derivate (z.B. Vitamin A, C, E, etc.), Selbstbräuner (z.B. DHA, Erythrulose, u.a.) sowie weitere kosmetische Wirkstoffe, wie z.B. Bisabolol, LPO, Ectoin, Emblica, Allantoin, Bioflavanoide und deren Derivate.

Organische UV-Filter werden in der Regel in einer Menge von 0,5 - 10 Gew.%, vorzugsweise 1-8 Gew.%, anorganische UV-Filter von 0,1 - 30 Gew.% eingesetzt, bezogen auf die kosmetische Formulierung.

Die Formulierungen können darüber hinaus weitere übliche hautschonende oder hautpflegende Wirkstoffe enthalten, wie z.B. Aloe Vera, Avocadoöl, Coenzym Q10, Grüner Tee Extrakt und auch Wirkstoffkomplexe.

Gegenstand der vorliegenden Erfindung sind ebenfalls Formulierungen, insbesondere kosmetische Formulierungen, die neben dem erfindungsgemäßen Pigmentgemisch mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Aloe Vera, Avocadoöl, Coenzym Q10, Grüner Tee Extrakt, Viskositätsreglern, Parfüm und Vitaminen enthalten.

Gegenstand der Erfindung ist auch die Verwendung des erfindungsgemäßen Pigmentgemisches in Farben, Lacken, Druckfarben, Sicherheitsdruckfarben, Kunststoffen, keramischen Materialien, Glasuren, Gläsern, als Tracer, in kosmetischen Formulierungen und zur Herstellung von Pigmentpräparationen und Trockenpräparaten.

Die nachfolgenden Beispiele sollen die Erfindung näher erläutern ohne sie jedoch zu beschränken.

### Beispiele

Die a- und b-Werte der einzelnen Interferenzpigmente werden mit dem Spektrometer ETA-Optik der Fa. STEAG wie folgt bestimmt: eine schwarz beschichtete Lackkarte wird mit einer Rakel (500 µm Spalthöhe) mit einem Nitrocellulose-Lack beschichtet, der 1,65 Gewichtsprozent des betreffenden Interferenzpigmentes bzw. der Pigmentmischung enthält. Nach dem Trocknen wird die Farbe mit dem oben genannten Meßgerät bei einem Einstrahlwinkel von 75° unter 95° gemessen. 90° ist die Senkrechte zur Lackkarte.

### Beispiel 1: Herstellung eines roten Interferenzpigmentes mit hohem Gelbanteil auf Basis von Aluminiumoxid-Plättchen

100 g Al₂O₃-Plättchen der Teilchengröße 5-30 µm (D₅₀ = 18 µm) werden in 1,6 | VE-Wasser suspendiert und die Suspension unter starkem Rühren auf 75 °C erwärmt. Zu dieser Suspension wird bei pH=2 eine Lösung aus 5 g SnCl₄ × 5H₂O in 70 g Wasser langsam zudosiert. Der pH-Wert wird mit 32 %iger Natronlauge konstant bei 2 gehalten. Danach wird der pH-Wert auf 1,8 gesenkt und bei diesem pH-Wert werden 820 g einer 32 %igen TiCl₄-Lösung langsam zudosiert. Dabei wird der pH-Wert mit 32 %iger Natronlauge konstant gehalten. Die Zugabe der TiCl₄-Lösung wird beim Erreichen des gewünschten Farbendpunktes beendet. Nach Beendigung der TiCl₄-Dosierung wird noch eine Stunde nachgerührt und dann das beschichtete Al₂O₃-Plättchen abfiltriert, gewaschen und bei 110 °C 15 h getrocknet. Zuletzt wird das Pigment 1 h bei 850 °C geglüht.

Das rote Interferenzpigment weist folgende a- und b-Werte auf:
a: 48,55 b: 18,37

### Beispiel 2: Herstellung eines roten Interferenzpigmentes mit hohem Gelbanteil auf Basis von Glimmer

100 g natürlicher Glimmer der Teilchengröße 5-25 µm (D₅₀ = 11 µm) werden in 2 l VE-Wasser suspendiert und die Suspension unter starkem Rühren auf 75 °C erwärmt. Zu dieser Suspension wird bei pH=2 eine Lösung aus 3 g SnCl₄ × 5H₂O in 100 g Wasser langsam zudosiert. Der pH-Wert wird mit 32 %iger Natronlauge konstant bei 2 gehalten. Danach wird der pH-Wert auf 1,8 gesenkt und bei diesem pH-Wert werden 1100 g einer 32 %igen TiCl₄-Lösung langsam zudosiert. Dabei wird der pH-Wert mit 32 %iger Natronlauge konstant gehalten. Die Zugabe der TiCl₄-Lösung wird beim Erreichen des gewünschten Farbendpunktes beendet. Nach Beendigung der TiCl₄-Dosierung wird noch eine Stunde nachgerührt und dann das beschichtete Glimmerpigment abfiltriert, gewaschen und bei 110 °C 15 h getrocknet. Zuletzt wird das Pigment 1 h bei 850°C geglüht.

Das rote Interferenzpigment weist folgende a- und b-Werte auf:
a: 40,64 b: 13,41

### Beispiel 3: Herstellung eines blauen Interferenzpigmentes auf Basis von Aluminiumoxid-Plättchen

100 g Al₂O₃-Plättchen der Teilchengröße 5-30 µm (D₅₀ = 18 µm) werden in 1,6 l VE-Wasser suspendiert und die Suspension unter starkem Rühren auf 75 °C erwärmt. Zu dieser Suspension wird bei pH=2 eine Lösung aus 9,3 g SnCl₄ × 5H₂O in 130 g Wasser langsam zudosiert. Der pH-Wert wird dabei mit 32 %iger Natronlauge konstant bei 2 gehalten. Danach wird der pH-Wert auf 1,8 gesenkt und bei diesem pH-Wert werden 714 g einer 32 %igen TiCl₄-Lösung langsam zudosiert. Dabei wird der pH-Wert mit 32 %iger Natronlauge konstant gehalten. Die Zugabe der TiCl₄-Lösung wird beim Erreichen des gewünschten Farbendpunktes beendet. Nach Beendigung der TiCl₄-Zugabe wird noch eine Stunde nachgerührt und dann das beschichtete Al₂O₃-Plättchen abfiltriert, gewaschen und bei 110 °C 15 h getrocknet. Zuletzt wird das Pigment 1 h bei 850 °C geglüht.

Das blaue Interferenzpigment weist folgende a- und b-Werte auf:
a: 2,61 b: -59,75

### Beispiel 4: Herstellung eines blauen Interferenzpigmentes auf Basis von Glimmer

70 g natürlicher Glimmer der Teilchengröße 1-20 µm (D₅₀ = 9 µm) werden in 1,4 l VE-Wasser suspendiert und die Suspension unter starkem Rühren auf 75°C erwärmt. Zu dieser Suspension wird bei pH=2 eine Lösung aus 15 g SnCl₄ × 5H₂O in 210 g Wasser langsam zudosiert. Der pH-Wert wird mit 32 %iger Natronlauge konstant bei 2 gehalten. Danach wird der pH-Wert auf 1,8 gesenkt und bei diesem pH-Wert werden 1056 g einer 32 %igen TiCl₄-Lösung langsam zudosiert. Dabei wird der pH-Wert mit 32 %iger Natronlauge konstant gehalten. Die Zugabe der TiCl₄-Lösung wird beim Erreichen des gewünschten Farbendpunktes beendet. Nach Beendigung der TiCl₄-Zugabe wird noch eine Stunde nachgerührt und dann das beschichtete Glimmerpigment abfiltriert, gewaschen und bei 110 °C 15 h getrocknet. Zuletzt wird das Pigment 1 h bei 850 °C geglüht.

Das blaue Interferenzpigment weist folgende a- und b-Werte auf:
a: 12,44 b: -82,11

### Beispiel 5: Herstellung eines grünen Interferenzpigmentes mit hohem Gelbanteil auf Basis von Aluminiumoxid-Plättchen

100 g Al₂O₃-Plättchen der Teilchengröße 5-30 µm (d₅₀ = 18 µm) werden in 1,6 l VE-Wasser suspendiert und die Suspension unter starkem Rühren auf 75 °C erwärmt. Zu dieser Suspension wird bei pH=2 eine Lösung aus 5 g SnCl₄ × 5H₂O in 100 g Wasser langsam zudosiert. Der pH-Wert wird mit 32 %iger Natronlauge konstant bei 2 gehalten. Danach wird der pH-Wert auf 1,8 gesenkt und bei diesem pH-Wert werden 878 g einer 32 %igen TiCl₄-Lösung langsam zudosiert. Dabei wird der pH-Wert mit 32 %iger Natronlauge konstant gehalten. Die Zugabe der TiCl₄-Lösung wird beim Erreichen des gewünschten Farbendpunktes beendet. Nach Beendigung der TiCl₄-Dosierung wird noch eine Stunde nachgerührt und dann das beschichtete Al₂O₃-Plättchen abfiltriert, gewaschen und bei 110 °C 15 h getrocknet. Zuletzt wird das Pigment 1 h bei 850 °C geglüht.

Das grüne Interferenzpigment weist folgende a- und b-Werte auf:
a: - 46,81 b: 34,62

### Beispiel 6: Herstellung eines grünen Interferenzpigmentes mit hohem Gelbanteil auf Basis von Glimmer

70 g natürlicher Glimmer der Teilchengröße 5-25 µm (D₅₀ = 11 µm) werden in 1,4 l VE-Wasser suspendiert und die Suspension unter starkem Rühren auf 75 °C erwärmt. Zu dieser Suspension wird bei pH=2 eine Lösung aus 2,1 g SnCl₄ × 5H₂O in 70 g Wasser langsam zudosiert. Der pH-Wert wird dabei mit 32%iger Natronlauge konstant bei 2 gehalten. Danach wird der pH-Wert auf 1,8 gesenkt und bei diesem pH-Wert werden 1005 g einer 32 %igen TiCl₄-Lösung langsam zudosiert. Die Zugabe der TiCl₄-Lösung wird beim Erreichen des gewünschten Farbendpunktes beendet. Nach Beendigung der TiCl₄-Dosierung wird noch eine Stunde nachgerührt und dann das beschichtete Glimmerpigment abfiltriert, gewaschen und bei 110 °C 15 h getrocknet. Zuletzt wird das Pigment 1 h bei 850 °C geglüht.

Das grüne Interferenzpigment weist folgende a- und b-Werte auf:
a: -32,89 b: 35,18

### Beispiel 7: Herstellung eines grünen Interferenzpigmentes mit hohem Gelbanteil auf Basis von Glas-Plättchen

100 g Glasplättchen der Teilchengröße 10-60 µm (D₅₀ = 22 µm) und einer mittleren Dicke von 450 nm werden in 1,4 l VE-Wasser suspendiert und die Suspension unter starkem Rühren auf 75 °C erwärmt. Nun wird der pH-Wert mit Natronlauge auf pH 8 erhöht und bei diesem pH-Wert im Verlauf von 90 min. eine Lösung von 38 g Natronwasserglas (13 % SiO₂) zudosiert. Dabei wird der pH-Wert mit Salzsäure (18 w-% HCl) konstant gehalten. Anschließend wird der pH-Wert innerhalb 45 min. auf pH 2 abgesenkt und bei diesem pH-Wert langsam eine Lösung von 5 g SnCl₄ × 5H₂O in 70 g Wasser zudosiert. Der pH-Wert wird dabei mit 32 %iger Natronlauge konstant bei 2 gehalten. Danach wird der pH-Wert auf 1,6 gesenkt und bei diesem pH-Wert werden 997 g einer 32 %igen TiCl₄-Lösung langsam zudosiert. Dabei wird der pH-Wert mit 32 %iger Natronlauge konstant gehalten. Die Zugabe der TiCl₄-Lösung wird beim Erreichen des gewünschten Farbendpunktes beendet. Nach Beendigung der TiCl₄-Dosierung wird noch eine Stunde nachgerührt und dann das beschichtete Glasplättchen abfiltriert, gewaschen und bei 110 °C 15 h getrocknet. Zuletzt wird das Pigment 1 h bei 650 °C geglüht.

Das grüne Interferenzpigment weist folgende a- und b-Werte auf:
a: -35,28 b: 37,11

### Beispiel 8: Herstellung eines roten Interferenzpigmentes mit hohem Gelbanteil auf Basis von synthetischem Glimmer

100 g synthetischer Glimmer der Teilchengröße 5-40 µm (D₅₀ = 17 µm) werden in 2 l VE-Wasser suspendiert und die Suspension unter starkem Rühren auf 75 °C erwärmt. Dann wird der pH-Wert mit Salzsäure (18 w-% HCl) auf pH 2,5 gestellt. Bei diesem pH-Wert werden 9 g AlCl₃-Lösung (29 w-% AlCl₃) innerhalb 2 min. zudosiert und 15 min. nachgerührt. Zu dieser Suspension werden bei pH=1,7 233 g SnCl₄-Lösung (2,1 w-% SnCl₄) langsam zudosiert. Der pH-Wert wird mit 32 %iger Natronlauge konstant bei 1,7 gehalten. Danach wird der pH-Wert mit Salzsäure auf 1,4 gesenkt und bei diesem pH-Wert werden 848 g einer 32 %igen TiCl₄-Lösung langsam zudosiert. Dabei wird der pH-Wert mit 32 %iger Natronlauge konstant gehalten. Die Zugabe der TiCl₄-Lösung wird beim Erreichen des gewünschten Farbendpunktes beendet. Nach Beendigung der TiCl₄-Dosierung wird noch eine Stunde nachgerührt und dann das beschichtete Glimmerpigment abfiltriert, gewaschen und bei 110 °C 15 h getrocknet. Zuletzt wird das Pigment 30 Min. bei 850 °C geglüht.

Das rote Interferenzpigment weist folgende a- und b-Werte auf:
a: 52,71 b: 17,24

### Farb-Beispiel 1:

Eine Mischung aus rotem Interferenzpigment gemäß Beispiel 2 und grünem Interferenzpigment gemäß Beispiel 6 im Mengenverhältnis 1 : 1 wird auf einer Lackkarte ausgezogen und vermessen. Die Mischung weist einen kräftigen Goldton auf.

Die Vermessung der Lackkarte ergibt folgende Werte:
a: 1,55 b: 22,13

### Anwendungsbeispiele

### Beispiel A1: Contouring Stift fürs Gesicht

| Ingredients | INCI (EU) | [%] |
|---|---|---|
| rotes Interferenz-pigment gemäß Beispiel 2 | Cl 77891 (Titanium Dioxide), Mica, Tin Oxide | 5,00 |
| grünes Interferenzpigment gemäß Beispiel 6 | Cl 77891 (Titanium Dioxide), Mica, Tin Oxide | 5,00 |
| blaues Interferenzpigment gemäß Beispiel 4 | Cl 77891 (Titanium Dioxide), Mica, Tin Oxide | 5,00 |
| Oxynex^{®} K liquid | PEG-8, TOCOPHEROL, ASCORBYL PALMITATE, ASCORBIC ACID, CITRIC ACID | 0,05 |
| Sensiva^{®} PA 20 | PHENETHYL ALCOHOL, ETHYLHEXYL GLYCERIN | 1,00 |
| Paraffin viscous | PARAFFINUM LIQUIDUM | 2,10 |
| Adeps Lanae | LANOLIN | 3,50 |
| Paracera C 44 | COPERNICIA CERIFERA CERA, CERESIN | 5,20 |
| Isopropyl Myristate | ISOPROPYL MYRISTATE | 5,60 |
| Wax white | CERA ALBA | 8,75 |
| Fragrance | PARFUM | 0,20 |
| Castor Oil | RICINUS COMMUNIS SEED OIL | Ad 100,00 |

### Beispiel A2: Medizinische Abdecksalbe zur starken Überdeckung von Pigment- / Altersflecken

| Ingredients | INCI (EU) | [%] |
|---|---|---|
| rotes Interferenz-pigment gemäß Beispiel 2 | Cl 77891 (Titanium Dioxide), Mica, Tin Oxide | 6,00 |
| grünes Interferenzpigment gemäß Beispiel 6 | Cl 77891 (Titanium Dioxide), Mica, Tin Oxide | 6,00 |
| blaues Interferenzpigment gemäß Beispiel 4 | Cl 77891 (Titanium Dioxide), Mica, Tin Oxide | 6,00 |
| Oxynex^{®} K liquid | PEG-8, TOCOPHEROL, ASCORBYL PALMITATE, ASCORBIC ACID, CITRIC ACID | 0,05 |
| Sensiva^{®} PA 20 | PHENETHYL ALCOHOL, ETHYLHEXYL GLYCERIN | 1,00 |
| Paraffin viscous | PARAFFINUM LIQUIDUM | 2,10 |
| Adeps Lanae | LANOLIN | 3,50 |
| Paracera C 44 | COPERNICIA CERIFERA CERA, CERESIN | 5,25 |
| Isopropyl Myristate | ISOPROPYL MYRISTATE | 5,60 |
| Wax white | CERA ALBA | 8,75 |
| Castor Oil | RICINUS COMMUNIS SEED OIL | Ad 100,00 |

### Beispiel A3: Gesichtsmakeup mit UV-Schutz und zur Auffrischung des Teints

| Ingredients | INCI (EU) | [%] |
|---|---|---|
| rotes Interferenzpigment gemäß Beispiel 2 | Cl 77891 (Titanium Dioxide), Mica, Tin Oxide | 7,50 |
| grünes Interferenzpigment gemäß Beispiel 6 | Cl 77891 (Titanium Dioxide), Mica, Tin Oxide | 2,50 |
| blaues Interferenzpigment gemäß Beispiel 4 | Cl 77891 (Titanium Dioxide), Mica, Tin Oxide | 5,00 |
| Texapon K 12 P | SODIUM LAURYL SULFATE | 0,60 |
| Veegum | MAGNESIUM ALUMINUM SILICATE | 1,00 |
| 1,2-Propanediol | PROPYLENE GLYCOL | 10,00 |
| Eusolex^{®} OCR | OCTOCRYLENE | 2,00 |
| Parteck^{®} LUB STA 50 | STEARIC ACID | 1,50 |
| Eusolex^{®} 9020 | BUTYL METHOXYDIBENZOYLMETHANE | 0,50 |
| RonaCare^{®} AP | BIS-ETHYLHEXYL HYDROXYDIMETHOXY BENZYLMALONATE | 0,50 |
| Parteck^{®} LUB MST | MAGNESIUM STEARATE | 0,10 |
| Tego Care 450 | POLYGLYCERYL-3 METHYLGLUCOSE DISTEARATE | 3,50 |
| Eutanol G | OCTYLDODECANOL | 5,50 |
| Isopropyl Myristate | ISOPROPYL MYRISTATE | 6,50 |
| Euxyl^{®} PE 9010 | PHENOXYETHANOL, ETHYLHEXYL GLYCERIN | 1,00 |
| Fragrance | PARFUM | 0,20 |
| Water, demineralized | AQUA | Ad 100,00 |

### Beispiel A4: Skin Elixir für eine leichte Farbauffrischung

| Ingredients | INCI (EU) | [%] |
|---|---|---|
| rotes Interferenzpigment gemäß Beispiel 2 | Cl 77891 (Titanium Dioxide), Mica, Tin Oxide | 1,00 |
| grünes Interferenzpigment gemäß Beispiel 6 | Cl 77891 (Titanium Dioxide), Mica, Tin Oxide | 2,00 |
| blaues Interferenzpigment gemäß Beispiel 4 | Cl 77891 (Titanium Dioxide), Mica, Tin Oxide | 0,50 |
| Glycerol 85% | GLYCERIN, AQUA | 3,00 |
| Carbopol^{®} ETD 2020 | ACRYLATES/C10-30 ALKYL ACRYLATE CROSSPOLYMER | 0,20 |
| Cetiol OE | DICAPRYLYL ETHER | 3,00 |
| Crodamol ISIS-LQ-(MV) | ISOSTEARYL ISOSTEARATE | 2,50 |
| Tegosoft^{®} DC | DECYL COCOATE | 1,50 |
| Novemer EC-2 | SODIUM | 0,50 |
| Polymer | ACRYLATES/BEHENETH-25 METHACRYLATE CROSSPOLYMER, HYDROGENATED POLYDECENE, LAURYL GLUCOSIDE | |
| Sodium Hydroxide, 10 % | AQUA, SODIUM HYDROXIDE | 0,00 |
| Water, demineralized | AQUA | Ad 100,00 |

### Beispiel A5: Liquid Foundation W/Si mit UV-Schutz

| Ingredients | INCI (EU) | [%] |
|---|---|---|
| rotes Interferenzpigment gemäß Beispiel 2 | CI 77891 (Titanium Dioxide), Mica, Tin Oxide | 3,00 |
| grünes Interferenzpigment gemäß Beispiel 6 | CI 77891 (Titanium Dioxide), Mica, Tin Oxide | 3,00 |
| blaues Interferenzpigment gemäß Beispiel 4 | CI 77891 (Titanium Dioxide), Mica, Tin Oxide | 3,00 |
| Glycerol 85% | GLYCERIN, AQUA | 8,00 |
| Eusolex^{®} T-S | TITANIUM DIOXIDE (NANO), ALUMINA, STEARIC ACID | 8,50 |
| KF-6028 | PEG-9 POL YDIMETHYLSILOXYETHYL DIMETHICONE | 3,50 |
| KF-6038 | LAURYL PEG-9 POL YDIMETHYLSILOXYETHYL DIMETHICONE | 2,00 |
| USG-105 | DIMETHICONE/VINYL DIMETHICONE CROSSPOLYMER, METHYL TRIMETHICONE | 1,00 |
| TMF-1.5 | METHYL TRIMETHICONE | 17,00 |
| DM-Fluid-A-6cs | DIMETHICONE | 10,00 |
| Salacos 913 | ISOTRIDECYL ISONONANOATE | 4,00 |
| KF-56A | DIPHENYLSILOXY PHENYL TRIMETHICONE | 5,00 |
| Bentone 38 V | DISTEARDIMONIUM HECTORITE | 0,85 |
| KSP-100 | VINYL DIMETHICONE/METHICONE SILSESQUIOXANE CROSSPOLYMER | 2,50 |
| Water, demineralized | AQUA | Ad 100,00 |

### Beispiel A6: Face Powder um leicht gelbliche Töne für blasse asiatische Haut auszubalancieren

| Ingredients | INCI (EU) | [%] |
|---|---|---|
| rotes Interferenzpigment gemäß Beispiel 2 | CI 77891 (Titanium Dioxide), Mica, Tin Oxide | 1,00 |
| blaues Interferenzpigment gemäß Beispiel 4 | CI 77891 (Titanium Dioxide), Mica, Tin Oxide | 1,50 |
| Unipure Red LC 381 | CI 77491 | 0,22 |
| Unipure Brown LC 889 | CI 77491, CI 77499 | 0,35 |
| Parteck^{®} LUB MST | MAGNESIUM STEARATE | 2,40 |
| Eutanol G | OCTYLDODECANOL | 4,40 |
| Parteck^{®} LUB Talc | TALC | Ad 100,00 |

### Beispiel A7: Face Powder um leicht gelbliche Töne auf dunkleren Hauttypen auszubalancieren

| Ingredients | INCI (EU) | [%] |
|---|---|---|
| rotes Interferenzpigment gemäß Beispiel 2 | CI 77891 (Titanium Dioxide), Mica, Tin Oxide | 2,50 |
| blaues Interferenzpigment gemäß Beispiel 4 | CI 77891 (Titanium Dioxide), Mica, Tin Oxide | 2,50 |
| Unipure Red LC 381 | CI 77491 | 0,22 |
| Unipure Brown LC 889 | CI 77491, CI 77499 | 0,35 |
| Parteck^{®} LUB MST | MAGNESIUM STEARATE | 2,40 |
| Eutanol G | OCTYLDODECANOL | 4,40 |
| Parteck^{®} LUB Talc | TALC | Ad 100,00 |

### Beispiel A8: Face Powder um leicht rötliche Töne auf kaukasischem Hauttyp auszubalancieren

| Ingredients | INCI (EU) | [%] |
|---|---|---|
| rotes Interferenzpigment gemäß Beispiel 2 | CI 77891 (Titanium Dioxide), Mica, Tin Oxide | 2,00 |
| blaues Interferenzpigment gemäß Beispiel 4 | CI 77891 (Titanium Dioxide), Mica, Tin Oxide | 0,50 |
| Unipure Red LC 381 | CI 77491 | 0,22 |
| Unipure Brown LC 889 | CI 77491, CI 77499 | 0,35 |
| Parteck^{®} LUB MST | MAGNESIUM STEARATE | 2,40 |
| Eutanol G | OCTYLDODECANOL | 4,40 |
| Parteck^{®} LUB Talc | TALC | Ad 100,00 |

## Patentansprüche

1. Pigmentgemisch enthaltend zwei oder drei Interferenzpigmente, die jeweils unterschiedliche Interferenzfarben aufweisen, wobei die Interferenzfarben ausgewählt sind aus den Farben Rot, Blau und Grün und die CIE-Lab*-Farbbereiche der Einzelpigmente wie folgt definiert sind:
| | |
|---|---|
| rotes Interferenzpigment: | a ≥ 25 und b ≥ 0 |
| blaues Interferenzpigment: | -20 ≤ a ≤ 40 und b ≤ -50 |
| grünes Interferenzpigment: | a ≤ -15 und b ≥ 0, |
und die a- und b-Werte der einzelnen Interferenzpigmente mit dem Spektrometer ETA-Optik der Fa. STEAG wie folgt bestimmt werden:
eine schwarz beschichtete Lackkarte wird mit einer Rakel (500 µm Spalthöhe) mit einem Nitrocellulose-Lack beschichtet, der 1,65 Gewichtsprozent des betreffenden Interferenzpigmentes bzw. der Pigmentmischung enthält; nach dem Trocknen wird die Farbe mit dem oben genannten Meßgerät bei einem Einstrahlwinkel von 75° unter 95° gemessen; 90° ist die Senkrechte zur Lackkarte.

2. Pigmentgemisch nach Anspruch 1, **dadurch gekennzeichnet, dass** die CIE-Lab*-Farbbereiche der Einzelpigmente wie folgt definiert sind:
| | |
|---|---|
| rotes Interferenzpigment: | a ≥ 30 und b ≥ 5 |
| blaues Interferenzpigment: | -15 ≤ a ≤ 30 und b ≤ -60 |
| grünes Interferenzpigment: | a ≤ -20 und b ≥ 15. |

3. Pigmentgemisch nach Anspruch 1 oder 2, **dadurch gekennzeichnet, dass** die CIE-Lab*-Farbbereiche der Einzelpigmente wie folgt definiert sind:
| | |
|---|---|
| rotes Interferenzpigment: | a ≥ 35 und b ≥ 5 |
| blaues Interferenzpigment: | -10 ≤ a ≤ 20 und b ≤ -65 |
| grünes Interferenzpigment: | a ≤ -25 und b ≥ 20. |

4. Pigmentgemisch nach einem oder mehreren der Ansprüche 1 bis 3, **dadurch gekennzeichnet, dass** die Interferenzpigmente auf plättchenförmigen Substraten basieren.

5. Pigmentgemisch nach einem oder mehreren der Ansprüche 1 bis 4, **dadurch gekennzeichnet, dass** die plättchenförmigen Substrate ausgewählt sind aus der Gruppe natürlicher Glimmer, synthetischer Glimmer, Talkum, Kaolin, Glasplättchen, SiO₂-Plättchen, Al₂O₃-Plättchen, Graphit-Plättchen, Fe₂O₃-Plättchen, BiOCI, TiO₂-Plättchen, Nitrid-Plättchen, Oxinitrid-, BN-Plättchen, Fischsilber oder deren Gemische.

6. Pigmentgemisch nach einem oder mehreren der Ansprüche 1 bis 5, **dadurch gekennzeichnet, dass** die plättchenförmigen Substrate ausgewählt sind aus der Gruppe natürlicher Glimmer, synthetischer Glimmer, Glasplättchen, SiO₂-Plättchen, Al₂O₃-Plättchen oder deren Gemische.

7. Pigmentgemisch nach einem oder mehreren der Ansprüche 1 bis 6, **dadurch gekennzeichnet, dass** es sich bei den plättchenförmigen Substraten um natürlichen und/oder synthetischen Glimmer handelt.

8. Pigmentgemisch nach einem oder mehreren der Ansprüche 1 bis 7, **dadurch gekennzeichnet, dass** die plättchenförmigen Substrate mit einer hochbrechenden Interferenzschicht umhüllt sind.

9. Pigmentgemisch nach einem oder mehreren der Ansprüche 1 bis 8, **dadurch gekennzeichnet, dass** die hochbrechende Schicht eine TiO₂-Schicht ist oder aus einer Schichtenfolge aus TiO₂ - SiO₂ - TiO₂ besteht.

10. Pigmentgemisch nach einem oder mehreren der Ansprüche 1 bis 9, **dadurch gekennzeichnet, dass** die Interferenzpigmente eine Teilchengröße von D₅₀ < 25 µm aufweisen.

11. Verfahren zur Herstellung des Pigmentgemisches nach einem oder mehreren der Ansprüche 1 bis 10, **dadurch gekennzeichnet, dass** zwei oder drei Interferenzpigmente miteinander gemischt werden.

12. Verwendung des Pigmentgemisches nach einem oder mehreren der Ansprüche 1 bis 10 in Farben, Lacken, Druckfarben, Sicherheitsdruckfarben, Kunststoffen, keramischen Materialien, Glasuren, Gläsern, in kosmetischen Formulierungen, und zur Herstellung von Pigmentpräparationen und Trockenpräparaten.

13. Verwendung des Pigmentgemisches nach Anspruch 12 in medizinischen Abdecksalben, Konturstiften, Make-ups, Foundations und Concealers.

14. Formulierungen enthaltend ein Pigmentgemisch nach einem oder mehreren der Ansprüche 1 bis 10.

15. Formulierungen nach Anspruch 14, **dadurch gekennzeichnet, dass** sie neben dem Pigmentgemisch mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Aloe Vera, Avocadoöl, Coenzym Q10, grüner Tee Extrakt, Viskositätsreglern, Parfüm und Vitaminen enthalten.

## Claims

1. Pigment mixture comprising two or three interference pigments which each have different interference colours, where the interference colours are selected from the colours red, blue and green and the CIE Lab* colour ranges of the individual pigments are defined as follows:
| | |
|---|---|
| red interference pigment: | a ≥ 25 and b ≥ 0 |
| blue interference pigment: | -20 ≤ a ≤ 40 and b ≤ -50 |
| green interference pigment: | a ≤ -15 and b ≥ 0, |
and the a and b values of the individual interference pigments are determined as follows using a STEAG ETA-Optik spectrometer: a black-coated paint card is coated with a nitrocellulose lacquer comprising 1.65 per cent by weight of the relevant interference pigment or pigment mixture using a hand coater (gap width 500 µm); after drying, the colour is measured at 95° using the above-mentioned measuring instrument with an angle of incidence of 75°; 90° is the perpendicular to the paint card.

2. Pigment mixture according to Claim 1, **characterised in that** the CIE Lab* colour ranges of the individual pigments are defined as follows:
| | |
|---|---|
| red interference pigment: | a ≥ 30 and b ≥ 5 |
| blue interference pigment: | -15 ≤ a ≤ 30 and b ≤ -60 |
| green interference pigment: | a ≤ -20 and b ≥ 15. |

3. Pigment mixture according to Claim 1 or 2, **characterised in that** the CIE Lab* colour ranges of the individual pigments are defined as follows:
| | |
|---|---|
| red interference pigment: | a ≥ 35 and b ≥ 5 |
| blue interference pigment: | -10 ≤ a ≤ 20 and b ≤ -65 |
| green interference pigment: | a ≤ -25 and b ≥ 20. |

4. Pigment mixture according to one or more of Claims 1 to 3, **characterised in that** the interference pigments are based on flake-form substrates.

5. Pigment mixture according to one or more of Claims 1 to 4, **characterised in that** the flake-form substrates are selected from the group natural mica, synthetic mica, talc, kaolin, glass flakes, SiO₂ flakes, Al₂O₃ flakes, graphite flakes, Fe₂O₃ flakes, BiOCI, TiO₂ flakes, nitride flakes, oxynitride flakes, BN flakes, pearl essence or mixtures thereof.

6. Pigment mixture according to one or more of Claims 1 to 5, **characterised in that** the flake-form substrates are selected from the group natural mica, synthetic mica, glass flakes, SiO₂ flakes, Al₂O₃ flakes or mixtures thereof.

7. Pigment mixture according to one or more of Claims 1 to 6, **characterised in that** the flake-form substrates are natural and/or synthetic mica.

8. Pigment mixture according to one or more of Claims 1 to 7, **characterised in that** the flake-form substrates are enveloped with a highly refractive interference layer.

9. Pigment mixture according to one or more of Claims 1 to 8, **characterised in that** the highly refractive layer is a TiO₂ layer or consists of a sequence of TiO₂ - SiO₂ - TiO₂.

10. Pigment mixture according to one or more of Claims 1 to 9, **characterised in that** the interference pigments have a particle size of D₅₀ < 25 µm.

11. Process for the preparation of the pigment mixture according to one or more of Claims 1 to 10, **characterised in that** two or three interference pigments are mixed with one another.

12. Use of the pigment mixture according to one or more of Claims 1 to 10 in paints, coatings, printing inks, security printing inks, plastics, ceramic materials, glazes, glasses, and cosmetic formulations, and for the preparation of pigment preparations and dry preparations.

13. Use of the pigment mixture according to Claim 12 in medical covering ointments, contour sticks, makeups, foundations and concealers.

14. Formulations comprising a pigment mixture according to one or more of Claims 1 to 10.

15. Formulations according to Claim 14, **characterised in that**, besides the pigment mixture, they comprise at least one constituent selected from the group of the absorbents, astringents, antimicrobial substances, antioxidants, antiperspirants, antifoaming agents, antidandruff active compounds, antistatics, binders, biological additives, bleaching agents, chelating agents, deodorising agents, emollients, emulsifiers, emulsion stabilisers, dyes, humectants, film formers, fillers, fragrances, flavours, insect repellents, preservatives, corrosion protection agents, cosmetic oils, solvents, oxidants, plant constituents, buffer substances, reducing agents, surfactants, propellant gases, opacifying agents, UV filters and UV absorbers, denaturing agents, aloe vera, avocado oil, coenzyme Q10, green tea extract, viscosity regulators, perfume and vitamins.

## Revendications

1. Mélange de pigments comprenant deux ou trois pigments d'interférence qui présentent chacun différentes couleurs d'interférence, où les couleurs d'interférence sont choisies parmi les couleurs rouge, bleu et vert et l'espace chromatique L*a*b* CIE des pigments individuels est défini comme suit :
| | |
|---|---|
| pigment d'interférence rouge : | a ≥ 25 et b ≥ 0 |
| pigment d'interférence bleu : | -20 ≤ a ≤ 40 et b ≤ -50 |
| pigment d'interférence vert : | a ≤ -15 et b ≥ 0, |
et les valeurs a et b des pigments d'interférence individuels sont déterminées comme suit à l'aide d'un spectromètre STEAG ETA-Optik : une carte de peinture revêtue de noir est enduite d'une laque nitrocellulo- sique comprenant 1,65 pour cent en poids du pigment d'interférence ou du mélange de pigments pertinent à l'aide d'une coucheuse manuelle (largeur de fente 500 µm) ; après séchage, la couleur est mesurée à 95° à l'aide de l'instrument de mesure susmentionné avec un angle d'incidence de 75° ; 90° est la perpendiculaire à la carte de peinture.

2. Mélange de pigments selon la revendication 1, **caractérisé en ce que** l'espace chromatique L*a*b* CIE des pigments individuels est défini comme suit :
| | |
|---|---|
| pigment d'interférence rouge : | a ≥ 30 et b ≥ 5 |
| pigment d'interférence bleu : | -15 ≤ a ≤ 30 et b ≤ -60 |
| pigment d'interférence vert : | a ≤ -20 et b ≥ 15. |

3. Mélange de pigments selon la revendication 1 ou 2, **caractérisé en ce que** l'espace chromatique L*a*b* CIE des pigments individuels est défini comme suit :
| | |
|---|---|
| pigment d'interférence rouge : | a ≥ 35 et b ≥ 5 |
| pigment d'interférence bleu : | -10 ≤ a ≤ 20 et b ≤ -65 |
| pigment d'interférence vert : | a ≤ -25 et b ≥ 20. |

4. Mélange de pigments selon l'une ou plusieurs parmi les revendications 1 à 3, **caractérisé en ce que** les pigments d'interférence sont à base de substrats en forme de paillettes.

5. Mélange de pigments selon l'une ou plusieurs parmi les revendications 1 à 4, **caractérisé en ce que** les substrats en forme de paillettes sont choisis dans le groupe constitué par le mica naturel, le mica synthétique, le talc, le kaolin, les paillettes de verres, les paillettes de SiO₂, les paillettes de Al₂O₃, les paillettes de graphite, les paillettes de Fe₂O₃, BiOCl, les paillettes de TiO₂, les paillettes de nitrure, les paillettes d'oxynitrure, les paillettes de BN, l'essence de perles ou des mélanges de ceux-ci.

6. Mélange de pigments selon l'une ou plusieurs parmi les revendications 1 à 5, **caractérisé en ce que** les substrats en forme de paillettes sont choisis dans le groupe constitué par le mica naturel, le mica synthétique, les paillettes de verres, les paillettes de SiO₂, les paillettes de Al₂O₃ ou des mélanges de ceux-ci.

7. Mélange de pigments selon l'une ou plusieurs parmi les revendications 1 à 6, **caractérisé en ce que** les substrats en forme de paillettes sont constitués de mica naturel et/ou synthétique.

8. Mélange de pigments selon l'une ou plusieurs parmi les revendications 1 à 7, **caractérisé en ce que** les substrats en forme de paillettes sont enveloppés par une couche d'interférence à haute réfraction.

9. Mélange de pigments selon l'une ou plusieurs parmi les revendications 1 à 8, **caractérisé en ce que** la couche à haute réfraction est une couche de TiO₂ ou est constituée d'une séquence de TiO₂ - SiO₂ - TiO₂.

10. Mélange de pigments selon l'une ou plusieurs parmi les revendications 1 à 9, **caractérisé en ce que** les pigments d'interférence présentent une taille de particule de D₅₀ < 25 µm.

11. Procédé de préparation du mélange de pigments selon l'une ou plusieurs parmi les revendications 1 à 10, **caractérisé en ce que** deux ou trois pigments d'interférence sont mélangés les uns avec les autres.

12. Utilisation du mélange de pigments selon l'une ou plusieurs parmi les revendications 1 à 10 dans les peintures, les revêtements, les encres d'impression, les encres d'impression de sécurité, les matières plastiques, les matériaux céramiques, les glaçures, les verres, et les formulations cosmétiques, et pour la préparation de préparations pigmentaires et de préparations sèches.

13. Utilisation du mélange de pigments selon la revendication 12, dans les pommades couvrantes médicales, les bâtons de contour, les maquillages, les fonds de teint et les correcteurs de teint.

14. Formulations comprenant un mélange de pigments selon l'une ou plusieurs parmi les revendications 1 à 10.

15. Formulations selon la revendication 14, **caractérisées en ce que**, outre le mélange de pigments, elles comprennent au moins un constituant choisi dans le groupe constitué par les absorbants, les astringents, les substances antimicrobiennes, les antioxydants, les antisudorifiques, les agents antimousse, les substances actives antipelliculaires, les antistatiques, les liants, les additifs biologiques, les agents de blanchiment, les agents chélatants, les agents désodorisants, les émollients, les émulsifiants, les stabilisateurs d'émulsion, les colorants, les humectants, les agents filmogènes, les charges, les fragrances, les arômes, les insectifuges, les conservateurs, les agents de protection contre la corrosion, les huiles cosmétiques, les solvants, les oxydants, les constituants végétaux, les substances tampons, les agents réducteurs, les agents tensioactifs, les gaz propulseurs, les agents opacifiants, les filtres anti-UV et les absorbeurs UV, les agents dénaturants, l'aloe vera, l'huile d'avocat, le coenzyme Q10, l'extrait de thé vert, les régulateurs de viscosité, le parfum et les vitamines.
